# EUROPÄISCHE PATENTANMELDUNG

(11) **EP 1 925 942 A1**
(43) Veröffentlichungstag der Anmeldung: **28.05.2008**
(21) Anmeldenummer: 07121333.4
(22) Anmeldetag: 22.11.2007
(51) Int. Cl.: G01N 33/66

(54) **Verfahren zur Diagnose des Diabetes mellitus**

(30) Priorität: 24.11.2006 DE 102006055604
(71) Anmelder: Schiffer, Roswitha, 520066 Aachen (DE)
(72) Erfinder: Keller, Ruprecht, Prof. Dr. Dr., 50939 Köln (DE)
(74) Vertreter: Savic, Bojan

(57) **Zusammenfassung**

Die vorliegende Erfindung betrifft ein Verfahren zur Diagnose eines Diabetes mellitus durch den Nachweis von mindestens zwei Polyolen, und gegebenenfalls zusätzlich einem oder mehreren Mono-, Di- und Oligosacchariden sowie Derivaten davon, in einer biologischen Probe, insbesondere in Körperflüssigkeiten, wie z.B. Blut, Serum, Plasma, Urin oder Liquor cerebrospinalis.

## Beschreibung

Die vorliegende Erfindung betrifft ein Verfahren zur Diagnose eines Diabetes mellitus durch den Nachweis von Polyolen in einer biologischen Probe, insbesondere Körperflüssigkeiten, wie z.B. Blut, Serum, Plasma, Urin oder Liquor cerebrospinalis.

### Hintergrund der Erfindung

Diabetes mellitus ist eine weit verbreitete Stoffwechselerkrankung, die allein in Deutschland rund sechs Millionen Menschen betrifft. Nach Herz-Kreislauf-Erkrankungen und Krebs ist diese Stoffwechselstörung die dritthäufigste Todesursache in den Industrieländern.

Diese Stoffwechselerkrankung ist auf eine Störung in der Signalübertragung durch das Polypeptidhormon Insulin zurückzuführen. Das führt dazu, dass bei einem Diabetes der Blutzuckerspiegel zwar erhöht ist, der Körper die Zucker aber nur ungenügend verwerten kann.

Das Polypeptidhormon Insulin wird in den Langerhans'schen Inseln oder Betazellen der Bauchspeicheldrüse (Pankreas) gebildet und wirkt hauptsächlich auf die Zellen der Muskulatur, der Leber und des Fettgewebes. Es stimuliert die Synthese von Glycogen, Fetten und Proteinen, während es den Abbau dieser Energielieferanten hemmt. Mit der Ausnahme von Gehirn- und Leberzellen, benötigen alle anderen Zelltypen Insulin, um Glucose aus der Blutbahn aufnehmen zu können. Im Zusammenspiel mit dem antagonistischen Hormon Glucagon ist Insulin für die Regulation der Blutglucosekonzentration verantwortlich. Die Insulinproduktion ist im Wesentlichen von der Nahrungsaufnahme abhängig - je mehr Zucker aufgenommen wird, desto mehr Insulin produziert der Körper.

Bei einem Diabetes wird entweder nur eine unzureichende Menge an Insulin sekretiert oder die Zielzellen werden durch das sekretierte Insulin nur unzureichend stimuliert. Als Folge steigt die Glucosekonzentration im Blut soweit an, dass Glucose mit dem Urin ausgeschieden wird.

Trotz der hohen Glucosekonzentration im Blut leiden die Zellen unter Glucosemangel, da die Aufnahme in die Zellen gestört ist. Da der Körper als Reaktion darauf andere Stoffwechselwege beschleunigt, z.B. die Hydrolyse von Triacylglyceriden, die β-Oxidation, die Gluconeogenese und die Bildung von Ketokörpern, kann sich als Folge eine Ketoazidose entwickeln. Dabei ist die Konzentration von Ketokörpern im Blut so stark erhöht, dass es zu einer Überlastung der Pufferkapazität von Blut und Nieren kommt. Die Nieren regulieren den pH-Wert des Blutes über die Ausscheidung von Protonen mit dem Urin. Die Exkretion der Protonen wird von der Ausscheidung von Natrium- und Kaliumionen, sowie Phosphat und Wasser begleitet, was eine Dehydratation hervorruft. Diese trägt zusätzlich negativ zum Wasserverlust durch den osmotischen Effekt der hohen Glucosekonzentration im Blut bei. Sowohl die Übersäuerung (Azidose) als auch die Dehydratation sind lebensbedrohlich.

Man unterscheidet in der Regel zwischen zwei Formen von Diabetes mellitus, die als Typ-1- und Typ-2-Diabetes bezeichnet werden.

Typ-1-Diabetes ist auch als insulinabhängiger oder juveniler Diabetes bekannt ist. Dieser tritt meist unvermittelt bereits im Jugendalter auf und ist von einer sich rasch verschlechternden Prognose begleitet. Bei dieser Form von Diabetes ist Insulin gar nicht oder fast nicht vorhanden, was auf das Fehlen oder einen Defekt in den Insulin-produzierenden Zellen des Pankreas zurückzuführen ist. Bei entsprechender genetischer Veranlagung wird dieser Zustand durch eine Autoimmunreaktion, die sich gegen die Langerhans'schen Inselzellen richtet, verursacht. Diese Autoimmunantwort kann durch Medikamente oder Viren ausgelöst werden und führt zur selektiven Zerstörung der insulinproduzierenden β-Zellen des Pankreas. Die Ursachen dafür sind weitgehend unbekannt, man weiß aber, dass bestimmte genetische Varianten des MHC-II Komplexes dabei eine Rolle spielen. Juvenile Diabetiker benötigen tägliche Insulininjektionen, müssen eine strenge Diät halten und körperliche Anstrengung vermeiden. Selbst dann ist ihre Lebenserwartung aufgrund degenerativer Folgeerkrankungen, wie z.B. Nieren- und Nervenschädigungen, kardiovaskulären Erkrankungen oder Erblindung verkürzt.

Typ-2-Diabetes wurde früher auch als Altersdiabetes bezeichnet, da er in der Regel nur bei älteren Menschen auftritt. Heute findet sich diese Form des Diabetes zunehmend auch bei übergewichtigen Kindern und Jugendlichen. Zwischen 80 und 90 Prozent der Typ-2-Diabetiker sind übergewichtig. Neben dem Übergewicht spielt aber auch eine genetische Prädisposition eine Rolle. Diese unterscheidet sich aber von der Veranlagung zur insulinabhängigen Diabetes. Fachleute rechnen damit, dass die Zahl der Typ-2-Diabetiker in den nächsten Jahren noch weiter steigt, da die Zahl an übergewichtigen Menschen immer weiter zunimmt und gleichzeitig das Durchschnittsalter weiter steigt.

Eine Vorstufe des Typ-2-Diabetes ist die sogenannte pathologische Glucosetoleranz. Dabei haben die Betroffenen eine normale oder sogar erhöhte Insulinkonzentration im Blut, aber der Körper kann die Glucose, die in ausreichender Menge vorhanden sind, nicht richtig verwerten. Die pathologische Glucosetoleranz ist häufig von Übergewicht, hohem Blutdruck, hohen Blutfettwerten und erhöhten Harnsäurewerten begleitet. Diese Symptome bzw. Erkrankungen werden unter dem Begriff "metabolisches Syndrom" zusammengefasst. Das metabolische Syndrom ist dabei aber nicht nur Vorstadium eines Diabetes mellitus, sondern bereits ein eigenes Krankheitsbild.

Beim Typ-2-Diabetes haben die Körperzellen einen gewissen Grad an Resistenz gegen Insulin entwickelt und werden durch normale Konzentration des Insulins nicht mehr ausreichend stimuliert. Die schwache Reaktion auf das Insulin führt zu einer verringerten Aufnahme der Glucose aus dem Blut, so dass in Folge davon die Glucosekonzentration im Blut erhöht ist.

Ursache für diese Insulinresistenz ist wahrscheinlich zuerst eine erhöhte Insulinproduktion als Folge erhöhter Nahrungszufuhr, dem damit verbundenem Übergewicht und Bewegungsmangel. Über einen gewissen Zeitraum reagieren die Zellen darauf mit einer allmählichen Unterdrückung der Synthese von Insulinrezeptoren, so dass eine Mangel an Insulinrezeptoren auf der Zelloberfläche entsteht. Als Reaktion darauf produziert die Bauchspeicheldrüse immer größere Mengen an Insulin, um die verminderte Insulinempfindlichkeit auszugleichen. Dies führt zunächst zu einer regelrechten Überlastung der Bauchspeicheldrüse und schließlich dazu, dass die Insulinproduktion versiegt und ein Insulinmangel eintritt.

Wie oben erwähnt spielen aber auch Erbfaktoren und Alter bei der Krankheitsentwicklung eine Rolle. Es ist bekannt, dass die Insulinproduktion mit zunehmendem Alter natürlicherweise abnimmt. Ist ein Elternteil an Typ-2-Diabetes erkrankt, werden die Kinder zu 25 bis 50 Prozent ebenfalls an Diabetes erkranken. Die persönlichen Lebensumstände sind aber für den Zeitpunkt des Krankheitsausbruchs ausschlaggebend. So führen Übergewicht und ungenügende körperliche Bewegung (Muskelarbeit) üblicherweise dazu, dass die Krankheit zu einem früheren Zeitpunkt ausbricht.

Rund 95% der Diabeteserkrankungen in Deutschland sind dem Typ-2 zuzuordnen, nur etwa 5% dem Typ-1.

Typ-2-Diabetes beginnt schleichend und wird oft erst sehr spät erkannt. Das liegt vor allem daran, dass Typ-2-Diabetiker in der Regel in der frühen Krankheitsphase nur unspezifische und wenig ausgeprägte Symptome zeigen. Daher wird die Diagnose Typ-2-Diabetes oft nur zufällig oder im Rahmen einer Folgeerkrankung, beispielsweise einem Herzinfarkt, gestellt. Die mild ausgeprägten Beschwerden umfassen hierbei Müdigkeit, häufige Haut- und Schleimhautinfektionen, Juckreiz, häufiges Wasserlassen (Polyurie) und starken Durst (Polydipsie). Auch Schwangerschaftsdiabetes oder die Einnahme bestimmter Medikamente (z.B. Kortisonpräparate) erhöhen das Risiko, an Diabetes zu erkranken. Wie bereits erwähnt, führen die milden Beschwerden dazu, dass Diabetes vom Typ-2 häufig erst sehr spät diagnostiziert wird, was die Prognose deutlich verschlechtert, da so bei der Erstdiagnose oftmals bereits Spätschäden oder Begleiterkrankungen vorliegen. Typische Spätschäden sind Retinopathie, Nephropathie, Neuropathie und Makroangiopathie (koronare Herzerkrankung und Apoplex, sowie arterielle Verschlusserkrankung), während die charakteristischen Begleiterkrankungen gestörte Wundheilung, kognitive Störungen jeder Art und Depressionen einschließen. Durch eine geeignete Therapie können diese Komplikationen im Regelfall hinausgezögert werden. Dies setzt aber wiederum eine frühe Diagnose des Diabetes mellitus voraus.

Die zu späte Diagnose des Diabetes führt neben der Verschlechterung der Prognose für den betroffenen Patienten auch zu gravierenden volkswirtschaftliche Nachteile, da 30% der unbehandelten Diabetes-Patienten eine diabetische Nephropathie entwickeln. Eine solche Nephropathie führt in der Regel nach 10 Jahren zu einer so weitgehenden Zerstörung der Nieren, dass die Patienten dialysiert werden müssen. Wegen der erheblichen Kosten einer Dialyse stellen diese Komplikationen bei weiter steigendem Durchschnittsalter der Bevölkerung für die nationalen Gesundheitssysteme der Industrieländer eine schwere Belastung dar, die in der Zukunft weiter ansteigen wird.

Aus diesem Grund ist die Frühdiagnose eines Diabetes mellitus von entscheidender Bedeutung.

Gegenwärtig wird zur Diabetes Diagnose die Glucosekonzentration im Urin mit Hilfe eines Teststreifens bestimmt. Lässt sich wiederholt Glucose im Urin nachweisen, ist das fast immer ein Hinweis auf das Vorliegen eines Diabetes. Für die Messung der Glucose im Urin existieren schon seit einiger Zeit Teststreifen, die so einfach angewendet werden können, dass auch ungeübte Patienten diese in einem Selbsttest verwenden können.

Allerdings hat die Messung der Glucosekonzentration im Urin eine maximale diagnostische Sensitivität von 50%, was eine große Zahl an falsch negativen Diagnosen bedingt.

Dieser Unsicherheit wird augenblicklich damit begegnet, dass von ärztlicher Seite auf konkreten Verdacht hin eine Messung des Blutzuckerspiegels durchgeführt wird. Bei der Messung von Glucose im Nüchternserum oder Plasma beträgt die diagnostische Sensitivität maximal 70%, so dass auch hier eine erhebliche Unsicherheit besteht.

In Zweifelsfällen folgt in der Regel ein oraler Glukose-Toleranz-Test. Hierbei wird dem Patienten eine große Menge Glucose oral gegeben und die Glucose-Konzentration im Blut, Serum oder Plasma in gewissen zeitlichen Abständen mehrmals bestimmt. Diese Methode gilt heute als Referenzmethode zur Diagnostik eines Diabetes mellitus. Sie ist aber wegen der zeitlichen Beanspruchung des Patienten und wegen der häufigen Messungen aufwendig und eignet sich deswegen nicht für ein Screening asymptomatischer Patienten, d.h. von Patienten bei denen kein konkreter Verdacht vorliegt.

Aus diesem Grund hat die Bestimmung der Glucose im Urin trotz der bekannten Mängel hinsichtlich der diagnostischen Sensitivität weiterhin einen sehr großen Stellenwert in der Diagnostik des Diabetes mellitus.

Bereits 1998 hatte Larner ein Verfahren zum Nachweis der Insulinresistenz, die im Frühstadium der Entstehung eines Diabetes auftritt, beschrieben, das auf dem Nachweis des Verhältnisses von Inositolderivaten im Urin basiert (US Patent Nummer 5,750,348). Dieses Verfahren hat allerdings den Nachteil, dass Inositol allein kein verlässlicher Indikator für das Vorliegen eines Diabetes mellitus ist.

Daher ist es das Ziel der vorliegenden Erfindung ist es, ein verbessertes diagnostisches Verfahren zur Diagnose eines Diabetes mellitus bereitzustellen, das sowohl eine verbesserte diagnostische Sensitivität aufweist und gleichzeitig die frühere Erkennung eines Diabetes erlaubt.

### Beschreibung der Erfindung

Bereits in der Frühphase der Entstehung eines Diabetes mellitus ändert sich in dem betroffenen Individuum der Zuckerstoffwechsel. Dieser veränderte Zuckerstoffwechsel zeichnet sich vor allem dadurch aus, dass aufgrund einer erhöhten Glucosekonzentration im Blut vermehrt alternative Stoffwechselwege aktiviert werden. Diese dienen dazu die erhöhte Glucosekonzentration durch Umsetzung der Glucose zu verringern. Diese alternativen Stoffwechselwege sind hochvariabel und führen zu einer Vielzahl von Endprodukten.

Die Erfinder der vorliegenden Erfindung haben herausgefunden, dass der veränderte Zuckerstoffwechsel die Aktivierung des Polyolzyklus einschließt. Über diesen Stoffwechselweg entstehen aus der Glucose eine Reihe verschiedener Polyole. Diese werden bereits im Frühstadium einer Störung des Zuckerstoffwechsels mit dem Urin ausgeschieden und können darin nachgewiesen werden. Während Glucose vom menschlichen Körper sehr gut rückresorbierbar ist, werden die Polyole weniger gut rückresorbiert und können daher im Allgemeinen bereits vor Glucose im Urin nachgewiesen werden. Dabei ist die Konzentration dieser Polyole im Urin ein sehr sensitiver Hinweis auf eine Störung im Zuckermetabolismus, welche sich in der Regel erst viel später als Diabetes manifestiert.

Gegenstand der vorliegenden Erfindung ist daher ein Verfahren zur Diagnose eines Diabetes mellitus durch den Nachweis von mindestens zwei Polyolen in einer biologischen Probe.

Mit "Polyolen" sind Polyalkohole gemeint, d.h. Kohlenwasserstoffe, vorzugsweise mit 3-10 Kohlenstoffatomen, die mehr als eine Hydroxylgruppe aber keine weitere funktionellen Gruppe, wie z.B. eine Aldehyd- oder Ketogruppe, tragen. Besonders bevorzugt sind Polyole die sich von Monosacchariden durch die Reduktion der Carbonylgruppe ableiten lassen. Beispiele für solche Polyole sind Adonitol (Ribitol) Arabitol, Dulcitol (Galacitol), Erythritol, Fucitol, Glucitol, Iditol, Inositol, Lactitol, Maltitol, Mannitol, Rhamnitol, Sorbitol, Threitol und Xylitol.

Der Ausdruck "Diabetes" oder "Diabetes mellitus" wird in der vorliegenden Erfindung als Bezeichnung für einen Diabetes vom Typ-2 verwendet.

In einer Ausführungsform der Erfindung ist die biologische Probe eine menschliche Probe, beispielsweise eine Körperflüssigkeit, die aus der Gruppe, die aus Blut, Serum, Plasma, Urin und Liquor cerebrospinalis besteht, ausgewählt wird. In einer Ausführungsform ist die biologische Probe Urin.

Das erfindungsgemäße Verfahren kann insbesondere auch dazu dienen mindestens zwei, d.h. beispielsweise 2, 3, 4, 5, 6, 7, 8, 9, 10 oder mehr Polyole in der Probe nachzuweisen. Eine Ausführungsform des erfindungsgemäßen Verfahrens schließt daher den unspezifischen Nachweis der Summe aller in der Probe vorhandener Polyole ein. Der Nachweis der mindestens zwei Polyole kann simultan als Summe oder sequentiell einzeln, d.h. spezifisch, erfolgen.

Im Zusammenhang der vorliegenden Erfindung bezieht sich der Ausdruck "unspezifisch" im Zusammenhang mit einem Nachweisverfahren auf den Nachweis einer Stoffgruppe bzw. der Gesamtkonzentration einer Stoffgruppe ohne die Einzelkonzentrationen der Substanzen, die unter diese Stoffgruppe fallen, zu bestimmen. Beispiele für Stoffgruppen, die im Rahmen dieser Erfindung von Bedeutung sind, sind beispielsweise Polyole oder Monosaccharide.

Der Nachweis der Polyole kann über ein enzymatisches Verfahren erfolgen. Dabei werden die Polyole mit einem oder mehreren geeigneten Enzymen umgesetzt, wobei eine nachweisbare Markersubstanz entsteht. Diese Markersubstanz kann das aus dem Polyol entstehende Produkt selbst oder ein Komplex aus den bei der enzymatischen Reaktion entstehenden Produkten und einem geeigneten Nachweisreagens sein.

In einer Ausführungsform der Erfindung ist das zum Nachweis der Polyole verwendete Enzym eine Dehydrogenase, beispielsweise eine Sorbitol Dehydrogenase (SDH). Für das Nachweisverfahren dieser Erfindung geeignete Sorbitol Dehydrogenasen sind beispielsweise pflanzlichen, tierischen oder bakteriellen Ursprungs. Eine geeignete pflanzliche SDH ist z.B. die aus Äpfeln (SDH aus Malus x domestica; EC 1.1.1.14), eine geeignete bakterielle Sorbitol Dehydrogenase stammt z.B. aus *Rhodobacter sphaeroides*.

Die Sorbitol Dehydrogenase verwendet NAD⁺ als Cofaktor und katalysiert als natürliche biochemische Stoffwechselreaktion die Umwandlung von Sorbitol in Fructose, wobei gleichzeitig NAD⁺ zu NADH und H+ reduziert wird. Sorbitol Dehydrogenase besitzt eine geringe Substratspezifität, so dass neben Sorbitol eine Reihe weiterer Polyole zu den entsprechenden Zuckern oxidiert werden können. Bei der Oxidation von Polyolen durch die SDH entsteht aus NAD⁺ NADH. Das entstandene NADH wird in einer Folgereaktion abgefangen und wieder zu NAD+ oxidiert. Diese Reaktion kann beispielsweise mit 3-(4,5-dimethylthiazol-2-yl)-2,5-diphenyltetrazoliumbromid (MTT) in Gegenwart des Enzyms NADH:dye-Dehydrogenase (NADH:dye-DH) = NADH: dye oxidoreductase (häufig fälschlicherweise auch als Diaphorase bezeichnet) nach folgendem Schema erfolgen:

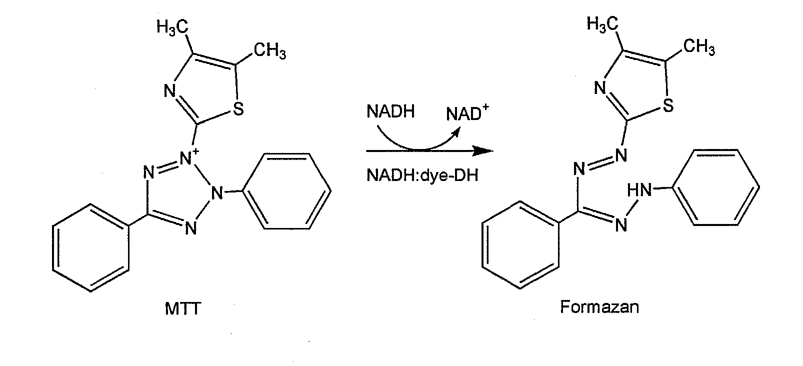

Nach dieser Reaktion steht NAD⁺ als Coenzym wieder zur Verfügung. Der Reaktionsverlauf kann spektrometrisch bei 578 nm verfolgt werden.

Der oben beschriebene enzymatische Nachweis der Polyole kann sowohl in einem nasschemischen Test, d.h. in Lösung, als auch mittels eines trägergebundenen Test, z.B. eines Teststäbchens, erfolgen. Der enzymatische Nachweis ist schnell und einfach und ist mittels eines Autoanalysers auch automatisierbar. Bei der Automatisierung kann ein sehr hoher Probendurchsatz erzielt werden, so dass das Bestimmungsverfahren weiter beschleunigt werden kann.

In einer anderen Ausführungsform der Erfindung wird die Konzentration der Polyole in einer biologischen Probe nicht als Summe, sondern einzeln sequentiell bestimmt. Verfahren die zu einem solchen Nachweis geeignet sind, schließen chromatographische Verfahren, beispielsweise Gaschromatographie und HPLC (high performance liquid chromatography), aber auch Kapillarelektrophorese ein. Diese Verfahren erlauben den Einzelnachweis von Polyolen in einer biologischen Probe. In Verbindung mit spektroskopischen Verfahren, zum Beispiel der Massenspektroskopie, kann nach einer entsprechenden Kalibrierung auch die zweifelsfreie Identifikation von spezifischen Polyolen erfolgen. Solche Verfahren erlauben ebenfalls die Bestimmung der Einzelkonzentrationen bestimmter Polyole.

In einer weiteren Ausführungsform der Erfindung werden zusätzlich zu den Polyolen in der biologischen Probe auch Zucker, insbesondere Mono- Di- und Oligosaccharide oder Derivate davon nachgewiesen.

Der Begriff "Zucker" bezieht sich in diesem Zusammenhang auf Mono-, Di- oder Oligosaccharide. Die Oligosaccharide die gemäß dem erfindungsgemäßen Verfahren bestimmt werden, sind vorzugsweise Tri-, Tetra-, Penta- oder Hexasaccharide, besonders bevorzugt Tri- oder Tetrasaccharide.

Mit "Monosaccharide" sind monomere Polyalkohole mit 3-8 Kohlenstoffatomen gemeint, die zusätzlich zu den Hydroxylgruppen auch eine Carbonylgruppe, d.h. Aldehyd- oder Ketogruppe, tragen. Beispiel für Monosaccharide, die im Rahmen der vorliegenden Erfindung bestimmt werden können schließen ein: Allose, Altrose, Arabinose, Fucose, Fructose, Galactose, Glucose, Gulose, Idose, Lyxose, Mannose, Psicose, Rhamnose, Ribose, Ribulose, Sorbose, Tagatose, Talose, Xylose und Xylulose.

"Disaccharide" bezieht auf ein Dimer aus Monosacchariden, die über eine glykosidische Bindung verknüpft sind. Beispiele für Disaccharide sind: Cellobiose, Lactulose, Lactose, Isomaltose, Maltose, Melibiose, Saccharose (Sucrose) und Trehalose.

"Oligosaccharide" bezieht sich auf ein Oligomer von Monosacchariden, das 3-10 Monosaccharideinheiten, die über glykosidische Bindungen verknüpft sind, umfasst. Insbesondere bezeichnet dieser Begriff im Rahmen der vorliegenden Erfindung Tri-, Tetra-, Penta- oder Hexasaccharide (3-6 Monosaccharideinheiten), vorzugsweise Tri- oder Tetrasaccharide. Zwei Beispiele für Oligosaccharide sind Raffinose (Trisaccharid) und Stachyose (Tetrasaccharid).

"Polysaccharide" bezieht sich im Zusammenhang der vorliegenden Erfindung auf Polymere aus mehr als 10 Monosacchariden, die über glykosidische Bindungen verknüpft sind.

Der Begriff "Derivate" bezieht sich im Zusammenhang mit Polyolen, Mono-, Di- und Oligosacchariden auf chemische Varianten der Polyole, Mono-, Di- und Oligosaccharide, die beispielsweise durch Oxidierung, Aminierung und N-Acetylierung erhalten werden können. Beispiele für solche Derivate sind: Galactosamin, Galacturonsäure, Gluconsäure, Glucosamin, Glucuronsäure, Mannuronsäure, N-Acetyl-Glucosamin, N-Acetyl-Muraminsäure und N-Acetyl-Neuraminsäure.

Die oben erwähnten Zucker können ebenfalls enzymatisch oder chromatographisch im Urin nachgewiesen werden. Geeignete Enzyme für einen enzymatischen Nachweis sind beispielsweise Monosaccharid-Oxidasen, die ebenfalls NAD⁺ als Coenzym verwenden. Beispiele für Monosaccharid-Oxigenasen sind Sorbitdehydrogenase und Glucoseoxidase. Die oben für den Nachweis von Polyolen beschriebenen chromatographischen Nachweisverfahren sind ebenfalls für den Nachweis von Mono-, Di- und Oligosacchariden sowie Derivaten davon geeignet.

In einer weiteren Ausführungsform der Erfindung werden neben der Konzentration der Polyole auch die Verhältnisse der Mengen verschiedener Zucker und Polyole zueinander und zu anderen Substanzen, die, wie z.B. Albumin und Kreatinin, im Urin auftreten, für die Diagnose herangezogen. Dabei werden die Quotienten aus den ermittelten Stoffkonzentrationen ausgewertet. Durch Vergleiche mit entsprechenden Referenzwerten, kann dann anhand der Ergebnisse die Diagnose eines Diabetes mellitus mit hoher Genauigkeit gestellt werden

Des Weiteren ist das erfindungsgemäße Verfahren auch für die Diagnose anderer Zuckerstoffwechselstörungen als Diabetes mellitus geeignet. Solche Zuckerstoffwechselstörungen können zu mikro- oder makrovaskulären, oder zentralnervösen oder peripher neuronalen Erkrankungen führen, daher ist eine frühere Diagnose solcher Erkrankungen mit dem erfindungsgemäßen Verfahren ebenfalls beabsichtigt.

### Beispiele

### Bestimmung von Polyolen und Monosacchariden im Urin mittels GCMS

Zur Vorreinigung der Probe werden 400 µl Urin mit 50 µl einer Lösung, die in Wasser gelöst einen oder mehrere Zucker, die nicht in nativem Urin vorkommen, enthält (interner Standard) und einer Spatelspitze Mischbettionentauscherharz versetzt und ca. 2 min geschüttelt. Anschließend wird zentrifugiert und 200 µl des Überstandes werden im Luftstrom bis zur Trockenheit eingedampft. Zu dem Rest werden 100µl BSTFA (N-O-Bis(trimethylsilyl)trifluoracetamid mit 2% TMS (Chlor-Trimethylsilan) gegeben. Die Mischung wird in einem geschlossenen Glasgefäß 10 min bei ca. 400 Watt mit Mikrowellen bestrahlt. Nach dem Abkühlen kann die flüssige Phase in die GC injiziert werden.

Die Analyse der Zucker erfolgt auf einem Gaschromatographen 5890 Serie II mit dem 5972 Massenselektiven Detektor der Firma Agilent (vormals Hewlett Packard).

Für die Trennung wird eine Rtx-1 Säule der Retek GmbH in Bad Soden (D) mit einer Länge von 15 m und einem Innendurchmesser von 10 µm verwendet. Die Säule wird nach der splitless Injektion einer 1 µl Probe mit 6°C pro Minute von 70°C auf 300°C erhitzt.

Die Zuordnung der Signale im Chromatogramm erfolgt durch den Vergleich der Massenspektren und Retentionszeiten mit Zuckerstandards. Die Quantifizierung wird berechnet aus den Intensitäten einer charakteristischen Masse im Vergleich zu Standards bekannter Einwaage und in Relation zu einem oder mehreren internen Standards. Als interne Standards werden Zucker wie z.B. Cellobiose, Melibiose oder Stachyose in einer Konzentration von 0,5 mg/ml verwendet.

### Enzymatische Bestimmung von Polyolen im Urin

Sorbitol Dehydrogenase (aus Malus x domesticus; EC 1.1.1.14) katalysiert in Gegenwart von NAD⁺ die Reaktion verschiedener Polyole zu den entsprechenden Monosacchariden unter Bildung von NADH und H⁺.

Lösung 1: Als Puffer wird eine 0,2 molare Tris (Tris(hydroxymethyl)aminomethan) Lösung pH 8.0 verwendet.
Lösung 2: 150 mg NAD⁺ werden in 2,5 ml Wasser gelöst.
Lösung 3: 50 mmol/l Tris pH 7.0
Enzymlösung: In 1 ml Lösung 3 werden 500 Einheiten (U bzw. Units) des Enzyms gelöst.

### Versuchsdurchführung

In einer Quarzglasküvette werden 1,5 ml Lösung 1 mit 5 µl Probe versetzt. Nach der Durchmischung werden 200 µl Lösung 2. Nun wird in einem UV-Spektrometer die Absorption bei 339 nm bestimmt. Nach dem Durchmischen mit 100 µl Enzymlösung wird das gebildete NADH dann mit 3-(4,5-dimethylthiazol-2-yl)-2,5-diphenyltetrazoliumbromid (MTT) in Gegenwart des Enzyms NADH:dye-Dehydrogenase (NADH:dye-DH) = NADH: dye oxidoreductase umgesetzt, wobei ein Farbstoff entsteht, der bei 578 nm gemessen werden kann.. Durch den Vergleich mit Standards bekannter Konzentration kann der Polyol-Gehalt in der Probe bestimmt werden.

### Klinische Daten

Tabelle 1 zeigt die über GC/MS bestimmten Konzentrationen verschiedener Polyole und Glucose (Glucose GC/MS) im Urin von Patienten ohne Proteinurie. Alle Patienten litten an einem über einen Glucosetoleranztest nachgewiesenen Diabetes mellitus. Als Vergleich ist ebenfalls die mittels eines üblichen enzymatischen Tests ermittelte Glucosekonzentration (Glucose enz.) angegeben. Dabei wird ein Summensignal der mittels GC/MS ermittelten Polyolkonzentrationen von > 0,02 mg/dl und eine enzymatisch bestimmte Glucosekonzentration > 16,0 mg/dl als positiv gewertet. Letzterer Wert entspricht der Konzentration, die im Stand der Technik üblicherweise als positiv bewertet wird.

Tabelle 1 zeigt die Ergebnisse einer GC/MS Untersuchung von Urinproben von 178 Patienten mit einem über einen oralen Glucosetoleranztest diagnostizierten Diabetes mellitus. 40 Patienten zeigten bereits eine Proteinurie aufgrund einer Nierenschädigung durch den zu spät diagnostizierten Diabetes mellitus. Mittels GC/MS wurden die Konzentrationen der Polyole Adonitol, Mannitol, Sorbitol und Xylitol im Urin bestimmt und ein Summensignal > 0,02 mg/dl als positiv bewertet. Die Glucosebestimmung im Urin erfolgte auf herkömmliche Art und Weise mit einem enzymatischen Test, wobei Glucosekonzentrationen > 16 mg/dl als positiv bewertet wurden.

**Tabelle 1. Ergebnisse einer Untersuchung von Urinproben von 178 Patienten mit anderweitig diagnostiziertem Diabetes mellitus.**

| | Gesamt | Proteinurie | Keine Proteinurie |
|---|---|---|---|
| Anzahl Proben | 178 | 40 | 138 |
| Glucose positiv | 21 (11,8%) | 4 (10,0%) | 17 (87,7%) |
| Polyol positiv | 141 (79,0%) | 21 (52,5%) | 120 (87,0%) |

Die Ergebnisse dieser Untersuchungen zeigen, dass sich mit dem erfindungsgemäßen Diagnoseverfahren eines Diabetes mellitus zur die Bestimmung der Konzentration von Polyolen im Urin eine größere Nachweisgenauigkeit des Diabetes mellitus ergab. Insgesamt konnte der übliche enzymatische Glucosenachweis den Diabetes mellitus in nur 11,8 % der Patienten, die an einem über einen oralen Glucosetoleranztest nachgewiesenen Diabetes mellitus litten, nachweisen. Dagegen konnte der erfindungsgemäße Polyolnachweis in 79,0 % der Patienten mit nachgewiesenem Diabetes mellitus den Diabetes mellitus nachweisen. Die Ergebnisse zeigen, dass der erfindungsgemäße Nachweis von Polyolen im Urin zur Diagnose eines Diabetes mellitus dem herkömmlichen Verfahren hinsichtlich der Genauigkeit und Verlässlichkeit deutlich überlegen ist.

## Patentansprüche

1. Verfahren zur Diagnose eines Diabetes mellitus, **dadurch gekennzeichnet, dass** das Verfahren den Nachweis von mindestens zwei Polyolen in einer biologischen Probe umfasst.

2. Verfahren nach Anspruch 1, **dadurch gekennzeichnet, dass** die biologische Probe eine menschliche Probe ist.

3. Verfahren nach Anspruch 1 oder 2, **dadurch gekennzeichnet, dass** die biologische Probe eine Körperflüssigkeit ist.

4. Verfahren nach Anspruch 3, **dadurch gekennzeichnet, dass** die Körperflüssigkeit aus der Gruppe Urin, Serum, Plasma, Vollblut und Liquor cerebrospinales ausgewählt wird.

5. Verfahren nach Anspruch 4, **dadurch gekennzeichnet, dass** die Körperflüssigkeit Urin ist.

6. Verfahren nach einem der vorhergehenden Ansprüche, **dadurch gekennzeichnet, dass** die Konzentration der Polyole gleichzeitig als Summe oder sequentiell einzeln bestimmt wird.

7. Verfahren nach einem der Ansprüche 1-6, **dadurch gekennzeichnet, dass** der Nachweis der Polyole enzymatisch erfolgt.

8. Verfahren nach Anspruch 7, **dadurch gekennzeichnet, dass** das Nachweisenzym eine Dehydrogenase ist.

9. Verfahren nach Anspruch 8, **dadurch gekennzeichnet, dass** die Dehydrogenase pflanzlichen, tierischen oder bakteriellen Ursprungs ist.

10. Verfahren nach Anspruch 8 oder 9, **dadurch gekennzeichnet, dass** das Nachweisenzym eine Sorbitol-Dehydrogenase ist.

11. Verfahren nach einem der Ansprüche 6 bis 10, **dadurch gekennzeichnet, dass** der Nachweis der Polyole in einem nasschemischen oder trägergebundenen Test erfolgt.

12. Verfahren nach Anspruch 1-6, **dadurch gekennzeichnet, dass** der Nachweis der Polyole in einem chromatographischen Verfahren erfolgt.

13. Verfahren nach Anspruch 12, **dadurch gekennzeichnet, dass** das chromatographische Verfahren Gaschromatographie oder HPLC ist.

14. Verfahren nach Anspruch 1-6, **dadurch gekennzeichnet, dass** der Nachweis der Polyole über Kapillarelektrophorese erfolgt.

15. Verfahren nach einem der Ansprüche 1-14, **dadurch gekennzeichnet, dass** die mindestens zwei Polyole unabhängig voneinander aus Adonitol (Ribitol) Arabitol, Dulcitol (Galacitol), Erythritol, Fucitol, Glucitol, Iditol, Inositol, Lactitol, Maltitol, Mannitol, Rhamnitol, Sorbitol, Threitol und Xylitol ausgewählt werden.

16. Verfahren nach Anspruch 15, **dadurch gekennzeichnet, dass** die nachgewiesenen Polyole Adonitol, Mannitol, Sorbitol und Xylitol sind.

17. Verfahren nach einem der Ansprüche 1-16, wobei zusätzlich zu den Polyolen in der biologischen Probe ein oder mehrere Mono-, Di-, oder Oligosaccharide, oder Derivate davon nachgewiesen werden.

18. Verfahren nach Anspruch 17, wobei die Mono-, Di-, oder Oligosaccharide, oder Derivate davon, aus der Gruppe, die aus Allose, Altrose, Arabinose, Cellobiose, Fucose, Fructose, Galactosamin, Galactose, Galacturonsäure, Gluconsäure, Glucosamin, Glucose, Glucuronsäure, Gulose, Idose, Isomaltose, Lactose, Lactulose, Lyxose, Maltose, Mannose, Mannuronsäure, Melibiose, N-Acetyl-Glucosamin, N-Acetyl-Muraminsäure, N-Acetyl-Neuraminsäure, Psicose, Raffinose, Rhamnose, Ribose, Ribulose, Saccharose (Sucrose), Sorbose, Stachyose, Tagatose, Talose, Trehalose, Xylose und Xylulose besteht, ausgewählt werden.

19. Verfahren nach einem der Ansprüche 17 oder 18, wobei der Nachweis von einem oder mehreren Mono-, Di-, oder Oligosacchariden, oder Derivaten davon enzymatisch erfolgt.

20. Verfahren nach Anspruch 19, wobei das Nachweisenzym eine Saccharidoxidase ist.

21. Verfahren nach Anspruch 20, wobei die Saccharidoxidase eine Monosaccharidoxidase ist.

22. Verfahren nach Anspruch 20 oder 21 wobei die Saccharidoxidase oder Monosaccharidoxidase pflanzlichen, tierischen oder bakteriellen Ursprungs ist.

23. Verfahren nach einem der Ansprüche 19-22, wobei das Nachweisenzym Glucoseoxidase ist.

24. Verfahren nach einem der Ansprüche 19 bis 23, wobei der Nachweis des einen oder der mehreren Mono-, Di-, oder Oligosaccharide oder Derivate davon in einem nasschemischen oder trägergebundenen Test erfolgt.

25. Verfahren nach Anspruch 17 oder 18, wobei der Nachweis des einen oder der mehreren Mono-, Di-, oder Oligosaccharide oder Derivaten davon in einem chromatographischen Verfahren erfolgt.

26. Verfahren nach Anspruch 25, wobei das chromatographische Verfahren Gaschromatographie oder HPLC ist.

27. Verfahren nach Anspruch 17 oder 18, wobei der Nachweis des einen oder der mehreren Mono-, Di-, oder Oligosaccharide oder Derivaten davon über Kapillarelektrophorese erfolgt.

28. Verfahren nach einem der Ansprüche 1 bis 27, **dadurch gekennzeichnet, dass** das Verfahren auch für die Diagnose anderer Zuckerstoffwechselstörungen als Diabetes mellitus geeignet ist.

29. Das Verfahren nach Anspruch 28, wobei die Zuckerstoffwechselstörungen zu mikro- oder makrovaskulären, zentralnervösen oder peripher neuronalen Erkrankungen führen.
